# FASCICULE DE BREVET EUROPEEN

(11) **EP 2 488 572 B1**
(45) Date de publication et mention de la délivrance du brevet: **10.05.2017**
(21) Numéro de dépôt: 10785128.9
(22) Date de dépôt: 18.10.2010
(51) Int. Cl.: C08J 3/205, C08J 3/20, C08J 3/11, C08K 5/00, C08K 3/00, C08K 5/13, B29C 67/00, A61K 8/88, A61K 9/14, A61K 47/34, A61Q 1/08, C08J 3/21, C08J 3/12, A61Q 1/10, C08K 5/01

(54) **PROCEDE DE PREPARATION DE POUDRE RECYCLABLE A BASE DE POLYAMIDE**
VERFAHREN ZUR HERSTELLUNG EINES RECYCELBAREN POLYAMIDPULVERS
METHOD FOR PREPARING A RECYCLABLE POLYAMIDE POWDER

(30) Priorité: 16.10.2009 FR 0957288
(43) Date de publication de la demande: 22.08.2012
(73) Titulaire: Arkema France, 92700 Colombes (FR)
(72) Inventeur: MATHIEU, Cyrille, F-76000 Rouen (FR); FILOU, Grégory, F-27500 Manneville Sur Risle (FR); LEMAITRE, Arnaud, F-27450 Saint-Martin Saint-Firmin (FR)
(86) Numéro de dépôt international: PCT/FR2010/052203
(87) Numéro de publication internationale: WO 2011/045550

(56) Documents cités:
- US-A- 4 334 056
- US-A- 5 763 512
- US-A1- 2004 106 691
- US-A1- 2006 041 041
- US-A1- 2006 071 359
- US-A1- 2009 075 081

## Description

La présente invention concerne un procédé de fabrication de poudre à base de polyamide, ladite poudre ayant des propriétés stables, c'est-à-dire ne variant pas en fonction de la température, et pouvant ainsi être recyclée.

La possibilité de recycler la poudre est notamment recherchée pour les procédés de fabrication d'objets par agglomération de poudre par fusion ou frittage provoqué par un rayonnement tel que par exemple un faisceau laser (laser sintering), un rayonnement infra rouge ou un rayonnement UV ou toute source de rayonnement électromagnétique. De tels procédés sont décrits dans les documents US6136948 et WO9606881. Par poudre recyclée, on entend une poudre ayant déjà été utilisée au moins une fois dans un procédé avant d'être réutilisée dans ce même procédé. Par poudre vierge, on entend une poudre neuve n'ayant jamais servi dans un procédé de fabrication d'objets.

Par poudres à base de polyamide, on entend les compositions pulvérulentes comprenant plus de 50 % en masse de polyamide (abrégé ci-après PA). Par poudres de polyamide, on entend celles comprenant plus de 95% en masse de polyamide. Les poudres de polyamide peuvent être produites par différents procédés qui conduisent chacun à des caractéristiques de poudre différentes. On peut citer les procédés de synthèse directs, qui conduisent par exemple à des poudres de polyamide 12 par polymérisation de lauryllactame ou d'acide amino dodécanoïque. En fonction du type de procédé, il est possible d'obtenir des poudres parfaitement sphériques non poreuses ou bien des poudres sphéroïdales poreuses. Dans ce dernier cas, on peut citer les poudres de PA 12, commercialisées par ARKEMA FRANCE sous le nom ORGASOL^{®}. Il existe aussi des procédés de dissolution/précipitation qui conduisent à des poudres de polymère par dissolution d'un polymère dans un solvant, puis reprécipitation sous forme de poudre. Selon leur procédé de fabrication, les poudres de polyamide obtenues sont plus ou moins blanches.

Le manque de stabilité sous certaines conditions (de température par exemple) de ces poudres de polyamide, notamment leur jaunissement, pose problème. Le recyclage de ces poudres devient alors limité, voire impossible. La couleur des pièces fabriquées avec une telle poudre n'est pas reproductible, puisque la couleur de la poudre utilisée varie au cours du temps. Pour les pièces fabriquées à partir de ces poudres, le même problème d'instabilité (de variation) de couleur peut apparaître progressivement au cours du temps.

Plusieurs méthodes ont été proposées pour résoudre ou au moins masquer ce problème d'instabilité de couleur des poudres de polyamide :
- l'ajout d'un pigment blanchissant (TiO₂ à une poudre de polyamide soit par compoundage puis broyage sous forme de poudre, soit par compoundage avant dissolution/précipitation, soit en début de procédé de dissolution/précipitation (EP1411087);
- l'ajout de sels métalliques d'acides monocarboxyliques, tels que des sels de sodium ou de calcium d'acides gras saturés, en particulier d'acide montanique (EP1424354), ou l'ajout d'un sel métallique d'acide faible et d'un dérivé d'acide gras, d'ester gras ou d'amide gras, tels que l'EBS ou éthylènebisstearylamide (EP1505108). L'ajout se fait :
   - soit par mélange à sec « dry blend » ou compoundage dans du PA fondu avant broyage pour former une poudre ;
   - soit par mélange de sels partiellement dissous dans un solvant et mélangés à une solution de polyamide (dissous ou en suspension) avant reprécipitation du polyamide (s'il est dissous) ou extraction du solvant (si le PA est en suspension) ;
   - soit par mélange de sels avec une solution de polyamide dissous dans l'éthanol avant précipitation complète du PA sous forme de poudre.
- l'ajout d'antioxydant(s) tel(s) que les antioxydants phénoliques et/ou phosphitiques par compoundage ou mélange à sec (dry blend) ;
- le lavage à l'éthanol de la poudre, pour éliminer les monomères résiduels, oligomères et autres impuretés ;
- l'addition de limiteurs de chaîne, de type mono- ou di-acides carboxyliques pendant la polymérisation pour obtenir des PA limités stables chimiquement lors de procédés laser sintering et de masse moléculaire stable (WO05097475 EP1413594).

Dans les documents précités, la diminution du jaunissement est seulement évoquée dans le document EP1411087, et elle n'est pas caractérisée mais uniquement corrélée à la stabilité des mesures DSC de la poudre ou des propriétés mécaniques (par exemple du module d'élasticité) des pièces obtenues à partir de la poudre. Les méthodes de stabilisation décrites ne permettent pas d'obtenir une poudre de couleur stable, capable de résister à plusieurs variations importantes de température. Il en résulte que la poudre ne peut pas être recyclée après une ou plusieurs variations de température sans variation de l'indice de jaune de la poudre. Par « plusieurs variations importantes de température » il faut comprendre au moins 3 cycles de chauffe successifs, de préférence au moins 5 cycles de chauffe successifs, jusqu'à une température inférieure de 2°C à 30°C par rapport à la température de fusion de la poudre.

La présente invention a donc pour but de fournir un procédé de fabrication d'une poudre de polyamide recyclable. Par poudre recyclable au sens de l'invention on entend une poudre stable thermiquement, donc qui ne jaunit pas, c'est-à-dire dont l'indice de jaune YI ne varie pas de plus de 2 unités selon la norme ASTM E 313-05, D1925, après plusieurs cycles de chauffe successifs (au moins 3 cycles) à une température proche de la température de fusion Tf de ladite poudre, c'est à dire à une température comprise dans la gamme allant de Tf-30°C à Tf-2°C.

La demanderesse a démontré que, de manière surprenante, l'addition sous certaines conditions d'un antioxydant à un polyamide sous forme pulvérulente, de pH compris dans la gamme allant de 3 à 8, et de préférence d'indice de jaune inférieur à 4, de préférence inférieur à -3, permettait de fabriquer une poudre recyclable conduisant à des objets de couleur identique, indépendamment du fait que la poudre selon l'invention soit vierge ou recyclée.

La présente invention a donc pour objet un procédé de préparation de poudre recyclable à base de polyamide, ledit procédé comprenant les étapes successives suivantes :
a) une étape d'ajout de 0,01 à 5%, de préférence de 0,01 à 1% en masse d'au moins un antioxydant sous forme pulvérulente à un mélange comprenant de 20 à 95% en masse de poudre à base de polyamide et de 5 à 80% en masse de liquide, sur la masse dudit mélange, ladite poudre de polyamide du mélange ayant un pH mesuré selon la norme ISO 787-9: 1981 compris dans la gamme allant de 3 à 8, de préférence de 4 à 7, ladite poudre à base de polyamide du mélange ayant de préférence un indice de jaune inférieur à 4 mesuré selon la norme ASTM E 313-05, D1925, ledit liquide étant non-solvant du polyamide à une température comprise entre 5°C et la température d'ébullition Teb dudit liquide et comprenant une coupe d'hydrocarbure;
b) une étape d'homogénéisation du mélange obtenu à l'étape a);
c) une étape de récupération de poudre isolée du liquide.

Avantageusement, la poudre, c'est-à-dire aussi bien la poudre initiale du mélange utilisée dans le procédé de l'invention que la poudre recyclable obtenue par le procédé, a un diamètre moyen en volume compris dans la gamme allant de 5 à 150 µm, de préférence de 20 à 100 µm.

La poudre utilisée dans le mélange de poudre et de liquide selon le procédé de l'invention a un pH compris dans la gamme allant de 3 à 8, de préférence de 4 à 7. Il est facile pour l'homme du métier d'adapter le pH de la poudre en ajoutant une quantité adaptée d'un système tampon ou d'une solution aqueuse d'un acide, par exemple d'acide hypophosphoreux, d'acide phosphorique, d'acide citrique ou de sels de ces acides. Des modes d'adaptation du pH de la poudre couramment utilisés sont par exemple décrits dans les documents EP1319681, US6281282, US2003114636.

Dans la présente description de l'invention, les mesures de pH sont effectuées selon la norme ISO 787-9 : 1981. On utilise, notamment dans les exemples ci-après, un pHmètre Consort 833 et une électrode de faible conductivité (Sonde Bioblock G90398) pour mesurer le pH de la poudre. On mesure en fait le pH d'une solution d'eau ultrapure après extraction des sels contenus dans la poudre. Pour cela, on mélange 5 g de poudre avec 100 ml d'eau ultra pure de pH 7 sous agitation (pendant 30 minutes), puis on mesure le pH de la solution.

La poudre utilisée dans le mélange de poudre et de liquide selon le procédé de l'invention a un indice de jaune inférieur à 4, de préférence inférieur à 3. De telles poudres d'indice de jaune inférieur à 3 sont commercialisées par la société Arkema, notamment sous la marque Orgasol®, Rilsan® et Pebax® et par Evonik, notamment sous la marque Vestosint®.

Avantageusement, ledit au moins un antioxydant est choisi parmi les antioxydants phénoliques, les phosphites et leurs mélanges.

A titre d'exemple d'antioxydant phénolique, on peut citer le (4,4'-Butylidenebis(2-t-butyl-5-methylphenol) commercialisé notamment sous le nom Lowinox 44B25 par Chemtura, le Pentaerythritol tetrakis(3-(3,5-di-tert-butyl-4-hydroxyphenyl)propionate) commercialisé notamment sous le nom Irganox® 1010 par Ciba, le N,N'-hexane-1,6-diylbis(3-(3,5-di-tert-butyl-4-hydroxyphenylpropionamide)) commercialisé notamment sous le nom Irganox® 1098 par Ciba, le 3,3',3',5,5',5'-hexa-tert-butyl-a,a',a'-(mesitylene-2,4,6-triyl)tri-p-cresol commercialisé notamment sous le nom Irganox® 1330 par Ciba, l'Ethylenebis(oxyethylene)bis-(3-(5-tert-butyl-4-hydroxy-m-tolyl)propionate) commercialisé notamment sous le nom Irganox® 245 par Ciba, 1,3,5-tris(3,5-di-tert-butyl-4-hydroxybenzyl)-1,3,5-triazine-2,4,6(1H,3H,5H)-trione commercialisé notamment sous le nom Irganox® 3114 par Ciba, le N'N'-(2 éthyl-2'éthoxyphényl)oxanilide commercialisé notamment sous le nom Tinuvin® 312 par Ciba, le Phénol 4,4',4"-trimethyl-1,3,5-benzenetriyl) tris-(methylene)] tris 2,6-bis(1,1-dimethylethyl) commercialisé notamment sous le nom Alvinox® 1330 par 3V, Hostanox 245 FF, Hostanox 245 Pwd, commercialisés par Clariant, le Pentaerythritol Tetrakis (3-(3,5-di-tert-butyl-4-hydroxyphenyl)propionate) commercialisé notamment sous les noms Evernox 10, Evernox 10GF, par Everspring Chemical Company Limited, l'Octadecyl-3-(3,5-di-tert-4-hydroxyphenyl)-propionate commercialisé notamment sous les noms Evernox 76, Evernox 76GF par Everspring Chemical Company Limited, le Tetrakis [Methylene-3(3',5'-di-tert-butyl-4-hydroxyphenyl) propionate] méthane commercialisé notamment sous le nom BNX® 1010 par Mayzo, Thiodiethylene bis[3-(3,5-di-tert-butyl-4-hydroxyphenyl)propionate] commercialisé notamment sous le nom BNX® 1035 par Mayzo, le Tetrakis [Methylene-3 (3',5'-di-tert-butyl-4-hydroxyphenyl)propionate] méthane, Octadecyl-3-(3,5-di-tert-butyl-4-hydroxyphenyl)-propionate commercialisé notamment sous le nom BNX® 2086 par Mayzo, le 1,3,5-tris(3,5-di-tert-butyl-4-hydroxybenzyl)-1,3,5-triazine-2,4,6(1H,3H,5H)trione commercialisé notamment sous le nom BNX® 3114 par Mayzo.

A titre d'exemple de phosphite (antioxydant phosphitique), on peut citer le Tris(2,4-di-tert-butylphenyl)phosphite commercialisé notamment sous les noms Irgafos® 168 par Ciba, CSFC 186 par China Scientific Fine Chemicals, Everfos 168 par Everspring Chemical Company Limited, et Benefos® 1680 par Mayzo.

A titre d'exemple d'antioxydant à la fois phénolique et phosphitique, on peut citer le mélange de tetrakismethylene(3,5-di-t-butyl-4-hydroxyhydrocinnamate)methane et tris(2,4-di-t-butylphenyl) phosphite commercialisé notamment sous le nom Anox BB 2777, Anox BB 2888, Anox BB 011, Anox BB 021 par Chemtura (Great Lakes), le mélange 20 % Stearyl-3-(3',5'-di-tert-butyl-4-hydroxyphenyl) propionate et 80% Tris (2,4- di-tert-butylphenyl) phosphite commercialisé notamment sous le nom BNX® 1900 par Mayzo.

Les antioxydants préférés pour la présente invention sont les antioxydants phénoliques car ils présentent l'avantage d'être stables, ne se dégradant pas sous une température proche de la température de fusion des poudres à base de polyamide. Par conséquent, ils ne laissent pas de résidus lorsqu'ils sont soumis à des températures proches de la température de fusion des poudres à base de polyamide, notamment lorsqu'ils sont utilisés dans une machine de frittage laser. Au contraire, il arrive que des antioxydants phosphitiques laissent des résidus qui encrassent la machine de frittage dans les parties de la machine qui ne sont pas en contact avec la poudre, notamment les parties optiques de la machine.

De préférence, l'étape a) d'ajout est réalisée à une température comprise dans la gamme allant de 15 à 105°C, de préférence de 50 à 90°C.

De préférence, l'étape b) d'homogénéisation comprend les étapes successives suivantes :
b1) une phase de chauffe dudit mélange obtenu à l'étape a) jusqu'à une température comprise dans la gamme allant de 50 à 120°C, de préférence de 60 à 90°C ;
b2) une phase isotherme chaude sous agitation, pendant laquelle la température du mélange est maintenue constante, comprise dans la gamme de températures allant de 50 à 120°C, de préférence de 60°C à 90°C pendant un temps suffisant pour homogénéiser la totalité du mélange.

L'étape c) de récupération de la poudre peut comprendre tout moyen de séparation solide/liquide (séparation poudre/liquide), par exemple par filtration, et/ou tout moyen de séchage de la poudre, notamment par évaporation du liquide.

Avantageusement, l'étape c) de récupération de poudre comprend les étapes successives suivantes :
c1) une phase de chauffe dudit mélange homogène obtenu à l'étape b) jusqu'à une température comprise entre la température d'ébullition dudit liquide et la température de fusion de la poudre ;
c2) une phase isotherme chaude pendant laquelle la température du mélange est maintenue constante comprise entre la température d'ébullition dudit liquide et la température de fusion de la poudre, pendant un temps suffisant pour permettre à la totalité du liquide de s'évaporer et obtenir une poudre recyclable selon l'invention.

A l'étape c), de manière alternative, additionnelle ou simultanée, la poudre est séchée sous dépression de manière à diminuer la température d'ébullition du liquide, et de sorte que l'étape c) est réalisée à une température plus basse, c'est-à-dire à une température de l'ordre de 80 à 100°C, par exemple à 90°C.

Avantageusement, ledit liquide du mélange a (à pression atmosphérique) une température d'ébullition Teb comprise dans la gamme allant de 70°C à 170°C, de préférence de 85°C à 170°C, de préférence de 120°C à 170°C. Ledit liquide comprend une coupe d'hydrocarbure, comme les isoparaffines, de préférence contenant de 6 à 12 atomes de carbone par molécule, et de point d'ébullition d'au moins 120°C. On peut citer notamment les mélanges d'isoparaffine, de N-paraffine et de cycloparaffine, dont la plage d'ébullition se situe entre 140 et 170°C.

La présente invention a également pour objet une poudre à base de polyamide de température de fusion Tf fabriquée selon le procédé de l'invention, ladite poudre ayant un pH compris dans la gamme allant de 3 à 8, de préférence de 4 à 7, et un indice de jaune YI stable (mesuré selon la norme ASTM E 313-05, D1925) ne variant pas de plus de 3 unités, de préférence ne variant pas de plus 2 unités, après au moins 3 cycles de chauffe, de préférence après au moins 5 cycles de chauffe successifs à une température comprise dans la gamme allant de Tf - 30°C à Tf - 2°C, ladite poudre comprenant de 0,01 à 5%, de préférence de 0,01 à 1% en masse d'au moins un antioxydant. Dans la présente description, la température de fusion Tf de la poudre correspond à la température de fusion en première chauffe de la poudre, mesurée selon la norme ISO 11357-3 Plastics - Differential scanning calorimetry (DSC) Part 3. De préférence, ladite poudre selon l'invention a un indice de jaune inférieur à 4, de préférence inférieur à 3.

Avantageusement, ladite poudre à base de polyamide selon l'invention comprend au moins un homopolyamide et/ou au moins un copolyamide et/ou au moins un copolyesteramide et/ou au moins un copolymère à blocs polyamide et blocs polyéther (abrégé PEBA) et/ou leurs mélanges.

Avantageusement, ladite poudre à base de polyamide comprend au moins un monomère choisi parmi les acides aminocarboxyliques, de préférence alpha,oméga-aminocarboxyliques, comprenant de 4 à 18 atomes de carbone, les couples diamine.diacide comprenant de 4 à 18 atomes de carbone, les lactames comprenant de 3 à 18 atomes de carbone, les lactones comprenant de 3 à 18 atomes de carbone et leurs mélanges.

Selon un mode de réalisation préféré de l'invention, lesdites particules à base de polyamide comprennent au moins un polyamide et/ou au moins un copolyamide et/ou au moins un copolyesteramide, et/ ou leurs mélanges.

Par polyamide (homopolyamide ou copolyamide) au sens de l'invention on entend les produits de condensation des lactames, des aminoacides et/ou des diacides avec les diamines et, en règle générale, tout polymère formé par des motifs ou monomères reliés entre eux par des groupes amides.

Par copolyesteramide, on entend les polymères issus de la copolymérisation de lactame(s) avec une ou plusieurs lactone(s) comme décrit dans le brevet EP1172396.

Le terme « monomère » dans la présente description des poudres à base de polyamides doit être pris au sens d' « unité répétitive ». Le cas où une unité répétitive du polyamide est constituée de l'association d'un diacide avec une diamine est particulier. On considère que c'est l'association d'une diamine et d'un diacide, c'est-à-dire le couple diamine.diacide (en quantité équimolaire), qui correspond au monomère. Ceci s'explique par le fait qu'individuellement, le diacide ou la diamine n'est qu'une unité structurale, qui ne suffit pas à elle seule à polymériser. Dans le cas où les particules de poudre selon l'invention comprenant au moins deux monomères différents, appelés «co-monomères», c'est à dire au moins un monomère et au moins un co-monomère (monomère différent du premier monomère), elles comprennent un copolymère tel qu'un copolyamide abrégé CoPA ou bien un coplyesteramide abrégé CoPEA.

A titre d'exemple de lactames, on peut citer ceux ayant de 3 à 12 atomes de carbone sur le cycle principal et pouvant être substitués. On peut citer par exemple le β,β-diméthylpropriolactame, le α,α-diméthylpropriolactame, l'amylolactame, le caprolactame, le capryllactame, l'oenantholactame, le 2-pyrrolidone et le lauryllactame.

A titre d'exemple de diacide (ou acide dicarboxylique), on peut citer les acides ayant entre 4 et 18 atomes de carbone. On peut citer par exemple, l'acide adipique, l'acide sébacique, l'acide azélaique, l'acide subérique, l'acide isophtalique, l'acide butanedioïque, l'acide 1,4 cyclohexyldicarboxylique, l'acide téréphtalique, le sel de sodium ou de lithium de l'acide sulphoisophtalique, les acides gras dimérisés(ces acides gras dimérisés ont une teneur en dimère d'au moins 98% et sont de préférence hydrogénés) et l'acide dodécanédioïque HOOC-(CH2)10-COOH.

A titre d'exemple de diamine, on peut citer les diamines aliphatiques ayant de 6 à 12 atomes, elle peut être arylique et/ou cyclique saturée. A titre d'exemples on peut citer l'hexaméthylènediamine, la pipérazine, la tetraméthylène diamine, l'octaméthylène diamine, la décaméthylène diamine, la dodécaméthylène diamine, le 1,5 diaminohexane, le 2,2,4-triméthyl-1,6-diamino-hexane, les polyols diamine, l'isophorone diamine (IPD), le méthyl pentaméthylènediamine (MPDM), la bis(aminocyclohéxyl) méthane (BACM), la bis(3-méthyl-4 aminocyclohéxyl) méthane (BMACM), la méthaxylyènediamine, le bis-p aminocyclohexylméthane et la triméthylhexaméthylène diamine.

A titre d'exemple d'aminoacide, on peut citer les alpha-oméga aminoacides, tels que les acides aminocaproïque, amino-7-heptanoïque, amino-11-undécanoïque, n-heptyl-11-aminoundécanoïque et amino-12-dodécanoïque.

A titre d'exemple de lactone, on peut citer la caprolactone, la valérolactone et la butyrolactone.

Le ou les monomère(s) préférentiellement utilisé(s) dans l'invention est ou sont choisi(s) parmi les lactames tels que par exemple le lauryllactame, le caprolactame, l'oenantholactame, le capryllactame ou leurs mélanges. De préférence, on utilise le lauryllactame seul ou en mélange avec le caprolactame.

La polymérisation entre les différents monomères précités peut être du type polycondensation hydrolytique, polymérisation anionique, ou encore polymérisation cationique. La polymérisation hydrolytique, surtout utilisée pour les lactames, est induite par l'eau à haute température. Par exemple, la polymérisation hydrolytique des lactames consiste à ouvrir le lactame par l'eau puis à chauffer sous pression pour polymériser. Eventuellement, un catalyseur tel que l'acide phosphorique peut également être employé dans le procédé hydrolytique. La polymérisation anionique s'effectue à des températures bien inférieures à celles appliquées pour les mécanismes hydrolytiques ou cationiques. La polymérisation anionique est conduite en continu ou bien de préférence, en discontinu (batch) dans un solvant. La voie anionique concerne plus spécifiquement les molécules cycliques, telles que les lactames et lactones. Par exemple, le mécanisme de polymérisation anionique des lactames se déroule en trois étapes : une étape d'initiation pour former l'anion lactamate, puis une réaction d'activation qui conduit à l'acyllactame et enfin l'étape de propagation. La méthode de polymérisation anionique est donc fondée essentiellement sur l'utilisation d'un catalyseur et d'un activateur en présence éventuellement d'une charge minérale ou organique finement divisée ayant un rôle de germe de cristallisation et en présence d'un amide. Le procédé est décrit dans les brevets EP192515 et EP303530. La polymérisation cationique est catalysée par des acides dans des conditions anhydres. Dans ce cas, des acides tels que l'acide chlorhydrique, l'acide phosphorique ou l'acide bromhydrique sont les plus réactifs mais l'utilisation d'acides de Lewis ou de sels d'ammoniums est également possible. Il existe essentiellement deux types d'activation et de croissance de la chaîne. Soit le monomère activé réagit avec le centre réactif neutre, soit c'est le centre réactif qui est activé et le monomère neutre.

De préférence, les poudres recyclables à base de polyamide de l'invention comprennent au moins un polyamide choisi parmi les polyamides et copolyamides comprenant au moins un des monomères suivants : 4.6, 4.T, 5.4, 5.9, 5.10, 5.12, 5.13, 5.14, 5.16, 5.18, 5.36, 6, 6.4, 6.9, 6.10, 6.12, 6.13, 6.14, 6.16, 6.18, 6.36, 6.T, 9, 10.4, 10.9, 10.10, 10.12, 10.13, 10.14, 10.16, 10.18, 10.36, 10.T, 11, 12, 12.4, 12.9, 12.10, 12.12, 12.13, 12.14, 12.16, 12.18, 12.36, 12.T, et leurs mélanges ; en particulier choisi parmi le PA 11, le PA 12, le PA 10.10, le PA 6, le PA 6/12, le PA 11/10.10, et leurs mélanges.

Selon un autre mode de réalisation avantageux de l'invention, lesdites particules à base de polyamide comprennent au moins un copolymère à blocs polyamide et blocs polyéther ou polyéther bloc amide, abrégé PEBA. Les PEBA résultent de la polycondensation de blocs polyamides à extrémités réactives avec des blocs polyéthers à extrémités réactives, telles que, entre autres :
1) blocs polyamides à bouts de chaîne diamines avec des blocs polyoxyalkylènes à bouts de chaînes dicarboxyliques.
2) blocs polyamides à bouts de chaînes dicarboxyliques avec des blocs polyoxyalkylènes à bouts de chaînes diamines, obtenues par cyanoéthylation et hydrogénation de blocs polyoxyalkylène alpha-oméga dihydroxylées aliphatiques appelées polyétherdiols
3) blocs polyamides à bouts de chaînes dicarboxyliques avec des polyétherdiols, les produits obtenus étant, dans ce cas particulier, des polyétheresteramides.

Les blocs polyamides à bouts de chaînes dicarboxyliques proviennent, par exemple, de la condensation de précurseurs de polyamides en présence d'un diacide carboxylique limiteur de chaîne. Les blocs polyamides à bouts de chaînes diamines proviennent par exemple de la condensation de précurseurs de polyamides en présence d'une diamine limiteur de chaîne. La masse molaire en nombre Mn des blocs polyamides est comprise dans la gamme allant de 400 à 20000 g/mol, de préférence de 500 à 10000 g/mol, et de préférence encore de 600 et 6000 g/mol.

Les polymères à blocs polyamides et blocs polyéthers peuvent aussi comprendre des motifs répartis de façon aléatoire.

Les blocs polyamides peuvent comporter des homopolyamides ou des copolyamides, tels que ceux décrits précédemment dans la présente description.

Par blocs polyéther (abrégé ci-après PE) au sens de l'invention, on entend les polyalkylènes éthers polyols, notamment des polyalkylènes éther diols. Les blocs polyéther (PE) comprennent au moins un polymère choisi parmi le poly(éthylène glycol) (PEG), le poly(1,2-propylène glycol) (PPG), le poly(1,3-propylène glycol) (PO3G), le poly(tétraméthylène glycol) (PTMG), le polyhexaméthylène glycol, le poly(1,3-propylène glycol) (PO3G), les poly(3-alkyl tétrahydrofurane) en particulier le poly(3-méthyltétrahydrofurane (poly(3MeTHF)), et leurs copolymères ou mélanges. On peut également envisager un bloc PE de type «copolyéther» à blocs ou statistique contenant un enchaînement d'au moins deux types de PE cités ci-dessus.

Les blocs polyéther peuvent également comprendre des blocs obtenus par oxyéthylation de bisphenols, tels que par exemple le bisphenol A. Ces derniers produits sont décrits dans le brevet EP 613 919.

Les blocs polyéthers peuvent aussi comprendre des amines primaires éthoxylées. On utilise avantageusement aussi ces blocs. A titre d'exemple d'amines primaires éthoxylées on peut citer les produits de formule : 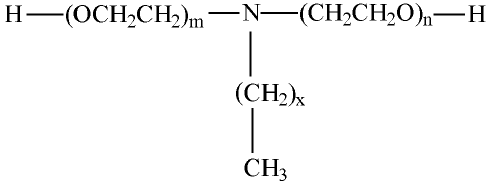 dans laquelle m et n sont compris entre 1 et 20 et x entre 8 et 18. Ces produits sont disponibles dans le commerce sous la marque NORAMOX® de la société CECA et sous la marque GENAMIN® de la société CLARIANT.

Ainsi, les fins de chaînes des blocs PE peuvent être diOH, diNH2, diisocyanate ou diacide suivant leur procédé de synthèse.

Les blocs PE à bouts de chaîne NH2, peuvent être obtenus par cyanoacétylation de séquences polyoxyalkylène alpha-oméga dihydroxylées aliphatiques appelées polétherdiols telles que les Jeffamines® D300, D400, D2000, ED-600, ED-900, ED2003, les Elastamines® RP-409, RP-2009, RT-1000, RE-600, RE-900, RE-2000, HT-1700, HE-180 de la société Huntsman. De tels blocs sont décrits dans les brevets JP 2004346274, JP 2004352794 et EP1482011. La masse molaire Mn des blocs polyéther est comprise dans la gamme allant de 100 à 6 000 g/mol et de préférence de 200 à 3 000 g/mol, encore plus préférentiellement de 250 à 2000 g/mol.

La préparation des copolymères à bloc(s) polyamide et bloc(s) polyéther selon l'invention comprend tout moyen permettant d'accrocher les blocs polyamide (bloc PA) et les blocs polyéther (bloc PE). En pratique, on utilise essentiellement deux procédés l'un dit en 2 étapes, l'autre en une étape, ces deux procédés sont bien connus et décrits par exemple dans la demande de brevet FR0856752.

Avantageusement, les poudres à base de polyamide de l'invention comprennent au moins un copolymère à blocs polyamide et blocs polyéther choisi parmi : PA12-PEG, PA6-PEG, PA6/12-PEG, PA11-PEG, PA12-PTMG, PA6-PTMG, PA6/12-PTMG, PA10.10-PEG, PA10.10-PTMG, PA11-PTMG, PA12-PEG/PPG, PA6-PEG/PPG, PA6/12-PEG/PPG, PA11-PEG/PPG, et leurs mélanges.

En fonction du mode de synthèse des polymères décrits ci-dessus, on obtient directement de la poudre ou alors des granulés. De la poudre est obtenue directement par polymérisation anionique. Pour obtenir de la poudre à base de polyamide dans le cas des autres types de polymérisation, on peut citer par exemple la dissolution-précipitation, c'est-à-dire solubilisation du polymère à base de polyamide dans un solvant à chaud puis précipitation de la poudre par refroidissement lent. Un tel procédé est décrit par exemple dans le document DE 2906647. On peut également citer l'atomisation, c'est-à-dire la pulvérisation d'une solution du polymère refroidi. Cette technique est également appelée « nébulisation à froid » ou « spray cooling ». Il existe aussi un procédé d'extrusion de polymère, suivi d'atomisation par une buse haute pression chauffée, puis refroidissement de la poudre obtenue. Cette technique est également appelée « nébulisation à chaud » ou « spray drying ». On peut encore citer le broyage/tamisage de granulés de polymère, éventuellement suivi d'une remontée en viscosité. Le broyage peut être cryogénique. Toutes ces techniques d'obtention de poudre sont déjà bien connues de l'homme du métier.

Avantageusement, lesdites particules de poudre à base de polyamide de l'invention ont un diamètre médian en volume compris dans la gamme allant de 5 à 150 µm, de préférence de 20 à 100 µm.

Avantageusement, ladite poudre est issue au moins partiellement de matières renouvelables ou bioressourcées, elle contient alors du ¹⁴C, cette teneur en biocarbone étant déterminée conformément à la norme ASTM D 6866.

Avantageusement, la poudre selon l'invention contient en outre au moins un additif choisi parmi : des agents azurants optiques, des pigments, des colorants, des anti-UV, des retardateurs de flamme, des stabilisants, des agents d'écoulement, des charges organiques ou minérales, de la poudre de silice, des liants de poudre, des nanotubes de carbone, et leurs mélanges.

La présente description divulgue également un procédé de fabrication d'objets par agglomération de poudre à base de polyamide par fusion en utilisant un rayonnement électromagnétique, la poudre à base de polyamide ayant été obtenue préalablement selon le procédé défini ci-dessus.

On dépose une fine couche de poudre de polyamide sur une plaque horizontale maintenue dans une enceinte chauffée à une température située entre la température de cristallisation Tc et la température de fusion Tf de la poudre de polyamide. Le laser agglomère des particules de poudre en différents points de la couche de poudre selon une géométrie correspondant à l'objet, par exemple à l'aide d'un ordinateur ayant en mémoire la forme de l'objet et restituant cette dernière sous forme de tranches. Ensuite, on abaisse la plaque horizontale d'une valeur correspondant à l'épaisseur d'une couche de poudre (par exemple entre 0,05 et 2 mm et généralement de l'ordre de 0,1 mm) puis on dépose une nouvelle couche de poudre et le laser agglomère des particules de poudre selon une géométrie correspondant à cette nouvelle tranche de l'objet et ainsi de suite. La procédure est répétée jusqu'à ce que l'on ait fabriqué tout l'objet. On obtient à l'intérieur de l'enceinte un objet entouré de poudre. Les parties qui n'ont pas été agglomérées sont donc restées à l'état de poudre. Ensuite on refroidit doucement l'ensemble et l'objet se solidifie dès que sa température descend en dessous de la température de cristallisation Tc. Après complet refroidissement, on sépare l'objet de la poudre qui peut être réutilisée, éventuellement mélangée à une quantité additionnelle de poudre vierge pour une autre opération. Avantageusement, la poudre selon l'invention peut être recyclée et réutilisée en tant que telle, sans l'ajout de poudre vierge. Dans le procédé de fabrication d'objets par agglomération de poudre par fusion selon l'invention, l'ajout d'un pourcentage de poudre vierge additionnel compris dans la gamme de 0 à 30% suffit amplement pour obtenir des objets aux couleurs et aux propriétés mécaniques stables, et reproductibles.

La présente description divulgue également un article manufacturé de couleur stable, ledit article étant obtenu par fusion à l'aide d'un rayonnement électromagnétique d'une poudre obtenue par le procédé conforme à l'invention.

Bien que, selon un mode de réalisation préféré, la poudre obtenue par le procédé selon l'invention soit particulièrement bien adaptée pour une utilisation dans les procédés de fabrication d'objets par fusion ou frittage provoqué par un rayonnement électromagnétique, l'utilisation de ladite poudre est bien entendu envisageable dans tout autre domaine qui requiert une poudre ayant les mêmes propriétés avantageuses de stabilité de couleur.

La présente description divulgue également l'utilisation de poudre telle que définie précédemment, dans des produits cosmétiques, pharmaceutiques ou de parfumerie. La présente description divulgue notamment une poudre cosmétique telle que définie précédemment, caractérisée en ce qu'elle constitue un fard à joues ou à paupières.

La présente description divulgue également l'utilisation de poudre obtenue par le procédé selon l'invention dans les revêtements, les composites, les adhésifs structuraux, les peintures, les compositions anticorrosion, les additifs pour papier, les technologies d'agglomération de poudre par fusion ou frittage provoqué par un rayonnement pour fabriquer des objets, les gels d'électrophorèse, les matériaux composites multicouches, l'industrie de l'emballage, l'ameublement, l'électroménager, les jouets, le textile, l'automobile et/ou l'électronique.

### Exemples

Les exemples ci-dessous illustrent la présente invention sans en limiter la portée. Dans les essais (exemples et comparatifs ci-dessous), sauf indication contraire, tous les pourcentages et parties sont exprimés en masse.

La poudre utilisée dans les essais ci-dessous est à base de polyamide 12 (PA 12) fabriqué selon le procédé décrit dans le document EP1814931. Son diamètre médian en volume est de 45,8 µm.

L'antioxydant utilisé dans les exemples ou comparatifs est un antioxydant phénolique, le Lowinox 44B25 (4,4'-Butylidenebis(2-t-butyl-5-methylphenol) commercialisé par Chemtura. L'antioxydant est parfois abrégé « AO » dans les tableaux ci-après.

Le liquide utilisé est un solvant Shellsol D25, également commercialisé sous le nom Essence 140/165, coupe pétrolière naphta légère hydrotraitée, de CAS N°64742-49-0.

Dans tous les essais ci-après, on mesure l'indice de jaune (abrégé YI) selon la norme ASTM E 313-05, D1925, utilisant l'illuminant de référence D65. On utilise un spectromètre Konica-Minolta CM-3600d. L'incertitude sur cette mesure est inférieure à 0,4. Ceci veut dire que si la différence entre deux valeurs de YI est supérieure à 0,4 alors les deux valeurs seront significativement différentes.

On mesure l'indice de jaune de poudres ayant subi de 0 à 3 (ou jusqu'à 5) cycles de chauffe à une température comprise entre Tf-30 et Tf-2 °C. Ces résultats sont reportés dans les tableaux 1 et 3.

On mesure l'indice de jaune de pièces (ici éprouvettes) fabriquées par frittage laser à partir de poudres ayant déjà subi de 0 à 3 (ou jusqu'à 5) cycles de chauffe à une température comprise entre Tf-30 et Tf-2 °C. Ces résultats sont reportés dans les tableaux 2 et 4.

Dans l'exemple 1 et les comparatifs 1, 2, et 3 suivants, on ajoute la même quantité d'antioxydant : soit 0,5% en masse d'antioxydant par rapport à la poudre sèche.

### Exemple 1 :

Le pH de la poudre de PA 12 est ajusté à 7 à l'aide d'une solution d'acide hypophosphoreux. L'indice de jaune de la poudre de PA 12 utilisée est inférieur à 4. On ajoute l'antioxydant à un mélange comprenant 90% en masse de poudre de PA 12 et 10% en masse de Shellsol D25.

### Comparatif 1 :

Le pH de la poudre de PA 12 est ajusté à 7 de la même façon que dans l'exemple 1, mais l'indice de jaune de la poudre utilisée est supérieur à 4. On mélange 0,5% d'antioxydant à cette poudre de PA 12 selon un procédé dry blend classique.

### Comparatif 2 :

Le pH de la poudre de PA 12 est ajusté à 7 de la même façon que dans l'exemple 1. L'indice de jaune de la poudre de PA 12 utilisée est inférieur à 4. On mélange 0,5% d'antioxydant à cette poudre de PA 12 selon un procédé dry blend classique.

### Comparatif 3 :

Le pH de la poudre de PA 12 n'est pas ajusté à 7, il est supérieur à 8. L'indice de jaune de la poudre de PA 12 utilisée est inférieur à 4. On ajoute l'antioxydant à un mélange comprenant 90% en masse de cette poudre de PA 12 et 10% en masse de Shellsol D25.

On mesure l'indice de jaune de ces poudres ayant subi de 0 à 3 cycles de chauffe à une température comprise entre Tf-30 et Tf-2 °C. Ces résultats sont reportés dans le tableau 1.

**Tableau 1**

| Essais | pH de la poudre | Antioxydant (%) / procédé d'ajout | Indice de jaune YI (norme E313-05) de la poudre: | | | | |
|---|---|---|---|---|---|---|---|
| | | | utilisée dans le procédé d'ajout d'antioxydant | avant phase de chauffe | après 1 phase de chauffe | après 2 phases de chauffe | après 3 phases de chauffe |
| Ex 1 | 3-8 | 0,5 / Inv | <4 | 1,2 | 2,2 | 2,8 | 2,8 |
| Comp 1 | 3-8 | 0,5 / DB | >4 | 4,3 | 6,8 | 6,9 | 7 |
| Comp 2 | 3-8 | 0,5 / DB | <4 | 2,2 | 3,7 | 4,2 | 4,6 |
| Comp 3 | >8 | 0,5 / Inv | <4 | 3,5 | 13,1 | | |

L'exemple 1 selon le procédé de l'invention permet d'obtenir une poudre recyclable, de faible indice de jaune (inférieur à 3) et dont l'indice de jaune n'augmente pas de plus de 2 unités, même après plusieurs recyclages, ici au moins 3 cycles de chauffe.

Les comparatifs 1 à 3 qui ne remplissent pas toutes les conditions du procédé d'ajout d'antioxydant selon l'invention, en utilisant :
- soit un polyamide de départ d'indice de jaune supérieur à 4 (Comp 1),
- soit un procédé de mélange par dry blend (DB) (Comp 2),
- soit un procédé d'ajout de l'antioxydant selon l'invention (Inv) excepté le fait que le polyamide du mélange est de pH non compris dans la gamme 3-8 (Comp 3).
Ces comparatifs 1 à 3 donnent une poudre finale qui est soit trop jaune (YI > 4) même avant un premier cycle de chauffe (Comp 3), soit qui jaunit très vite dès les premiers cycles de chauffe (Comp 1 et 2 : variation de YI supérieure à 2 après 3 cycles de chauffe).

On fabrique dans le même temps par frittage laser (« laser sintering », parfois abrégé « LS » dans les tableaux) des articles 3D, ici des éprouvettes de dimension conformes à la norme ISO 527 1B, à partir de poudre de l'exemple 1 ayant subi respectivement 1, 2 et 3 phases de chauffe dans une machine de frittage laser de marque Formiga P100 EOS, c'est à dire respectivement :
- à partir de poudre vierge qui subit une phase de chauffe supérieure à la température de fusion Tf de la poudre dans la machine pour fabriquer l'éprouvette,
- à partir de poudre recyclée qui a déjà subi 1 phase de chauffe à une température de Tf-30°C à Tf-2°C et qui subit une dernière phase de chauffe à une température supérieure à la température de fusion Tf de la poudre pour fabriquer l'éprouvette.
- à partir de poudre recyclée qui a déjà subi 2 phases de chauffe à une température de Tf-30°C à Tf-2°C et qui subit une dernière phase de chauffe à une température supérieure à la température de fusion Tf de la poudre pour fabriquer l'éprouvette.

Pour réaliser ces essais, on n'a pas eu besoin d'ajouter de poudre vierge à la poudre recyclée dans la machine LS après chaque cycle de frittage, car il restait suffisamment de poudre recyclée pour la fabrication de la pièce suivante (le cycle de frittage suivant). Grâce à la poudre recyclable selon l'invention, un pourcentage de poudre vierge additionnel compris dans la gamme de 0 à 30 % est suffisant. Dans les essais décrits ici, il est de 0%.

**Tableau 2**

| | Indice de jaune YI (norme E313-65) de la pièce fabriquée à partir de la poudre ayant subi: | | |
|---|---|---|---|
| | 1 phase de chauffe poudre vierge | 2 phases de chauffe poudre recyclée | 3 phases de chauffe poudre recyclée |
| Pièce fabriquée à partir de poudre de l'Exemple 1 | 1,4 | 2,6 | 2,9 |

Les éprouvettes obtenues avec la poudre de l'exemple 1 ont une couleur stable, leur indice de jaune restant inférieur à 3 et ne variant pas de plus de 2 unités entre chaque éprouvette fabriquée successivement avec de la poudre de l'exemple 1 de plus en plus recyclée (jusqu'à 3 phases de chauffe) .

On compare ensuite l'indice de jaune de poudres de l'exemple 2 suivant (selon l'invention) avec celui des poudres des comparatifs 2 et 4 (tableau 3) ; puis l'indice de jaune de pièces obtenues à partir de ces poudres (tableau 4 ci-dessous) :

### Exemple 2 :

Le pH de la poudre de PA 12 fabriqué selon le procédé décrit dans le document EP1814931 est ajusté à 7 à l'aide d'une solution d'acide hypophosphoreux. L'indice de jaune de la poudre de PA 12 est inférieur à 4. La poudre de polyamide 12 est chargée dans un sécheur dans lequel a déjà été mis l'antioxydant (Lowinox 44B25) selon une quantité massique correspondant à 0,5% par rapport à la poudre sèche ou 0,4% par rapport au mélange de poudre et de liquide (Shellsol D25) selon le procédé de l'invention. La charge du sécheur est de 48 kg de poudre essorée avec un extrait sec résiduel de 89,5%, ce qui correspond à un mélange selon le procédé de l'invention (un mélange comprenant de 20 à 95% en masse de poudre à base de polyamide et de 5 à 80% en masse de liquide). La masse d'antioxydant Lowinox 44B25 chargée au préalable dans le sécheur est de 215 g.

Sous agitation, la température du mélange est montée à 80°C puis maintenue à 80°C pendant 1 heure. Après ce palier, sans arrêter ni l'agitation ni la chauffe, on procède au séchage final :
- La température du mélange est montée à 95°C et dans le même temps, le sécheur est mis sous vide à 50 mbar.
- On suit le séchage en pesant les distillats recueillis dans un fût installé sur une balance.
- On arrête le séchage quand le poids du fût est stabilisé pendant 1 h.

Le sécheur est remis à pression atmosphérique et à température ambiante puis vidangé. On obtient une poudre de polyamide 12 recyclable, stabilisée selon le procédé de l'invention.

### Comparatif 4 :

Le pH de la poudre de PA 12 est ajusté à 7 de la même façon que dans l'exemple 2. L'indice de jaune de la poudre de PA 12 utilisée est inférieur à 4. Mais dans cet essais, on n'ajoute pas d'antioxydant.

### Comparatif 2 :

Le pH de la poudre de PA 12 est ajusté à 7 de la même façon que dans l'exemple 2. L'indice de jaune de la poudre de PA 12 utilisée est inférieur à 4, il est de 1,7.

1,5kg d'antioxydant Lowinox 44B25 sont ajoutés à 300kg de cette poudre de PA 12 dans un flobin.

Le mélange obtenu est homogénéisé à température ambiante pendant 4 heures par rotation du flobin sur lui-même (dry blend).

Remarque : le mélange obtenu au comparatif 2 présente une homogénéisation satisfaisante : le produit soumis à une température en four de 174°C sous oxygène ne présente pas de points d'oxydation colorés, contrairement à de la poudre non additivée (comparatif 4).

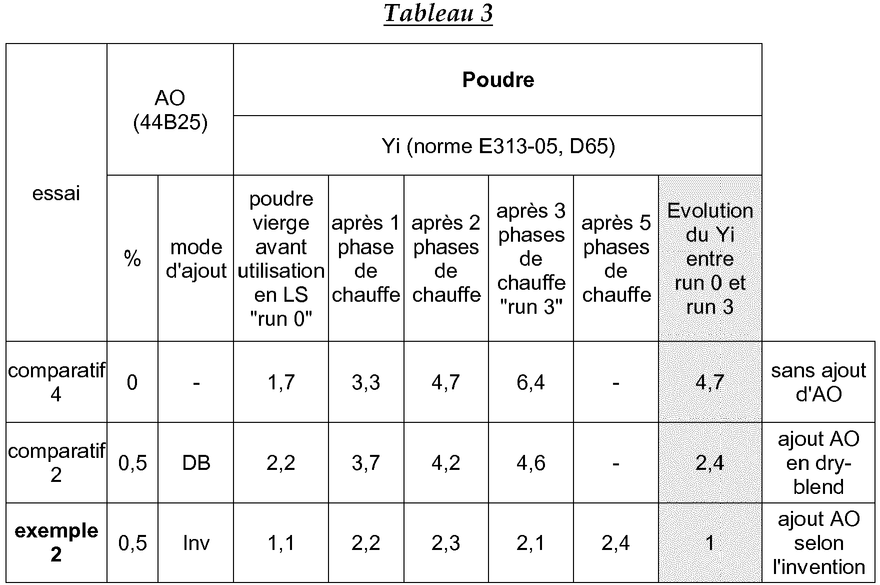

Comme visible sur le tableau 3, contrairement aux comparatifs 2 et 4, l'exemple 2 selon le procédé de l'invention permet d'obtenir une poudre recyclable, de faible indice de jaune (inférieur à 3) et dont l'indice de jaune n'augmente pas de plus de 2 unités, même après plusieurs recyclages, ici après au moins 5 cycles de chauffe dans le tableau 3.

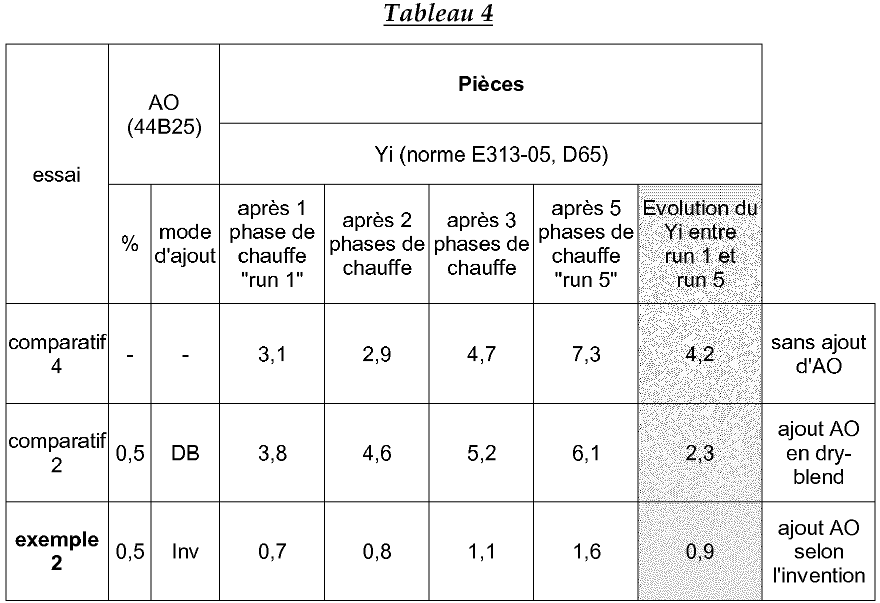

Comme visible sur le tableau 4, contrairement aux éprouvettes obtenues avec la poudre des comparatifs 2 et 4, les éprouvettes obtenues avec la poudre de l'exemple 2 ont une couleur stable, leur indice de jaune reste ici inférieur à 3 et ne varie pas de plus de 2 unités entre chaque éprouvette fabriquée successivement avec de la poudre de l'exemple 2, de plus en plus recyclée, ici après au moins 5 cycles de chauffe dans le tableau 4.

## Revendications

1. Procédé de préparation de poudre recyclable à base de polyamide, ledit procédé comprenant les étapes successives suivantes :
a) une étape d'ajout de 0,01 à 5% en masse d'au moins un antioxydant sous forme pulvérulente à un mélange comprenant de 20 à 95% en masse de poudre à base de polyamide et de 5 à 80% en masse de liquide sur la masse dudit mélange, ladite poudre à base de polyamide du mélange ayant un pH mesuré selon la norme ISO 787-9 : 1981 compris dans la gamme allant de 3 à 8 et un indice de jaune inférieur à 4 mesuré selon la norme ASTM E 313-05, D1925, ledit liquide étant non-solvant du polyamide à une température comprise entre 5°C et la température d'ébullition Teb dudit liquide et comprenant une coupe d'hydrocarbure ;
b) une étape d'homogénéisation du mélange obtenu à l'étape a);
c) une étape de récupération de poudre isolée du liquide.

2. Procédé selon la revendication 1, dans lequel la poudre recyclable a un diamètre moyen en volume compris dans la gamme allant de 5 à 150 µm.

3. Procédé selon la revendication 1 ou 2, dans lequel l'étape a) d'ajout est réalisée à une température comprise dans la gamme allant de 15 à 105°C, de préférence de 50 à 90°C.

4. Procédé selon l'une quelconque des revendications 1 à 3 dans lequel ledit au moins un antioxydant est choisi parmi les antioxydants phénoliques, les phosphites et leurs mélanges.

5. Procédé selon l'une quelconque des revendications précédentes, dans lequel l'étape b) d'homogénéisation comprend les étapes successives suivantes :
b1) une phase de chauffe dudit mélange obtenu à l'étape a) jusqu'à une température comprise dans la gamme allant de 50 à 120°C, de préférence de 60 à 90°C;
b2) une phase isotherme chaude sous agitation, pendant laquelle la température du mélange est maintenue constante, comprise dans la gamme de températures allant de 50 à 120°C, de préférence de 60 à 90°C pendant un temps suffisant pour homogénéiser la totalité du mélange;

6. Procédé selon l'une quelconque des revendications précédentes, dans lequel l'étape c) de récupération de poudre comprend les étapes successives suivantes :
c1) une phase de chauffe dudit mélange homogène obtenu à l'étape b) jusqu'à une température comprise entre la température d'ébullition dudit liquide et la température de fusion de la poudre ;
c2) une phase isotherme chaude pendant laquelle la température du mélange est maintenue constante comprise entre la température d'ébullition dudit liquide et la température de fusion de la poudre, pendant un temps suffisant pour permettre à la totalité du liquide de s'évaporer et obtenir une poudre.

7. Procédé selon l'une quelconque des revendications précédentes, dans lequel ladite poudre à base de polyamide comprend au moins un monomère choisi parmi les acides aminocarboxyliques, de préférence alpha,oméga-aminocarboxyliques, comprenant de 4 à 18 atomes de carbone, les couples diamine.diacide comprenant de 4 à 18 atomes de carbone, les lactames comprenant de 3 à 18 atomes de carbone, les lactones comprenant de 3 à 18 atomes de carbone et leurs mélanges.

8. Procédé selon l'une quelconque des revendications précédentes, dans lequel ledit liquide a une température d'ébullition Teb comprise dans la gamme allant de 70°C à 170°C.

## Patentansprüche

1. Verfahren zur Herstellung eines recycelbaren Pulvers auf Basis von Polyamid, wobei das Verfahren nacheinander die folgenden Schritte umfasst:
a) einen Schritt des Hinzufügens von 0,01 bis 5 Massen-% mindestens eines Antioxidans in pulverförmiger Form zu einem Gemisch, umfassend 20 bis 95 Massen-% Pulver auf Basis von Polyamid und 5 bis 80 Massen-% einer Flüssigkeit auf der Masse des Gemisches, wobei das Pulver auf Basis von Polyamid des Gemisches einen pH-Wert, gemessen nach der Norm ISO 787-9:1981, der in dem Bereich von 3 bis 8 liegt, und einen Gelbindex unter 4, gemessen nach der Norm ASTM E 313-05, D1925, aufweist, wobei die Flüssigkeit bei einer Temperatur zwischen 5 °C und der Siedetemperatur Teb der Flüssigkeit ein Nicht-Lösungsmittel des Polyamids ist und einen Kohlenwasserstoffschnitt umfasst;
b) einen Schritt der Homogenisierung des in Schritt a) erhaltenen Gemisches;
c) einen Schritt der Rückgewinnung von Pulver durch Abtrennung von der Flüssigkeit.

2. Verfahren nach Anspruch 1, bei dem das recycelbare Pulver einen durchschnittlichen Volumendurchmesser in dem Bereich von 5 bis 150 µm hat.

3. Verfahren nach Anspruch 1 oder 2, bei dem der Schritt a) des Hinzufügens bei einer Temperatur in dem Bereich von 15 bis 105 °C, vorzugsweise von 50 bis 90 °C, erfolgt.

4. Verfahren nach einem der Ansprüche 1 bis 3, bei dem das mindestens eine Antioxidans unter den Phenol-Antioxidantien, den Phosphiten und ihren Gemischen ausgewählt ist.

5. Verfahren nach einem der vorhergehenden Ansprüche, bei dem der Schritt b) der Homogenisierung nacheinander die folgenden Schritte umfasst:
b1) eine Phase der Erhitzung des in Schritt a) erhaltenen Gemisches auf eine Temperatur in dem Bereich von 50 bis 120 °C, vorzugsweise von 60 bis 90 °C;
b2) eine heiße isotherme Phase mit Schütteln, während der die Temperatur des Gemisches konstant gehalten wird und sich in dem Temperaturbereich von 50 bis 120 °C, vorzugsweise von 60 bis 90 °C, während einer ausreichenden Zeit befindet, um die Gesamtheit des Gemisches zu homogenisieren.

6. Verfahren nach einem der vorhergehenden Ansprüche, bei dem der Schritt c) der Rückgewinnung von Pulver nacheinander die folgenden Schritte umfasst:
c1) eine Phase der Erhitzung des in Schritt b) erhaltenen homogenen Gemisches auf eine Temperatur zwischen der Siedetemperatur der Flüssigkeit und der Schmelztemperatur des Pulvers;
c2) eine heiße isotherme Phase, während der die Temperatur des Gemisches zwischen der Siedetemperatur der Flüssigkeit und der Schmelztemperatur des Pulvers während einer ausreichenden Zeit konstant gehalten wird, um es der Gesamtheit der Flüssigkeit zu ermöglichen zu verdampfen, und um ein Pulver zu erhalten.

7. Verfahren nach einem der vorhergehenden Ansprüche, bei dem das Pulver auf Basis von Polyamid mindestens ein Monomer umfasst, das ausgewählt ist unter den Aminocarbonsäuren, vorzugsweise den Alpha,Omega-Aminocarbonsäuren, umfassend 4 bis 18 Kohlenstoffatome, wobei die Diamin-Disäure-Paare 4 bis 18 Kohlenstoffatome umfassen, wobei die Lactame 3 bis 18 Kohlenstoffatome umfassen, wobei die Lactone 3 bis 18 Kohlenstoffatome umfassen, und ihren Gemischen.

8. Verfahren nach einem der vorhergehenden Ansprüche, bei dem die Flüssigkeit eine Siedetemperatur Teb in dem Bereich von 70 °C bis 170 °C hat.

## Claims

1. A process for the preparation of a recyclable polyamide-based powder, said process comprising the following successive stages:
a) a stage of addition of from 0.01 to 5% by weight of at least one antioxidant in the pulverulent form to a mixture comprising from 20 to 95% by weight of polyamide-based powder and from 5 to 80% by weight of liquid, with regard to the weight of said mixture, said polyamide-based powder of the mixture having a pH according to standard ISO 787-9: 1981 within the range extending from 3 to 8 and a yellowness index of less than 4, measured according to standard ASTM E 313-05, D1925, said liquid being a nonsolvent for the polyamide at a temperature of between 5°C and the boiling point B.p. of said liquid and comprising a hydrocarbon fraction;
b) a stage of homogenization of the mixture obtained in stage a);
c) a stage of recovery of powder isolated from the liquid.

2. The process as claimed in claim 1, in which the recyclable powder has a mean diameter by volume within the range extending from 5 to 150 µm.

3. The process as claimed in claim 1 or 2, in which the addition stage a) is carried out at a temperature within the range extending from 15 to 105°C, preferably from 50 to 90°C.

4. The process as claimed in any one of claims 1 to 3, in which said at least one antioxidant is chosen from phenolic antioxidants, phosphites and their mixtures.

5. The process as claimed in any one of the preceding claims, in which the homogenization stage b) comprises the following successive stages:
b1) a phase of heating said mixture obtained in stage a) up to a temperature within the range extending from 50 to 120°C, preferably from 60 to 90°C;
b2) a hot isothermal stage with stirring, during which the temperature of the mixture is kept constant, within the range of temperatures extending from 50 to 120°C, preferably from 60 to 90°C, for a time sufficient to homogenize the entire mixture.

6. The process as claimed in any one of the preceding claims, in which the stage c) of recovery of powder comprises the following successive stages:
c1) a phase of heating said homogeneous mixture obtained in stage b) up to a temperature between the boiling point of said liquid and the melting point of the powder;
c2) a hot isothermal phase during which the temperature of the mixture is kept constant, between the boiling point of said liquid and the melting point of the power, for a time sufficient to make it possible for all of the liquid to evaporate and to obtain a powder.

7. The process as claimed in any one of the preceding claims, in which said polyamide-based powder comprises at least one monomer chosen from aminocarboxylic acids, preferably α,ω-aminocarboxylic acids, comprising from 4 to 18 carbon atoms, diamine.diacid pairs comprising from 4 to 18 carbon atoms, lactams comprising from 3 to 18 carbon atoms, lactones comprising from 3 to 18 carbon atoms and their mixtures.

8. The process as claimed in any one of the preceding claims, in which said liquid has a boiling point B.p. within the range extending from 70 to 170°C.
